# EUROPEAN PATENT APPLICATION

(11) **EP 0 543 551 A1**
(43) Date of publication of application: **26.05.1993**
(21) Application number: 92310256.0
(22) Date of filing: 10.11.1992
(51) Int. Cl.: A61B 5/06

(54) **Inductively coupled RF tracking system for use in invasive imaging of a living body**

(30) Priority: 18.11.1991 US 793923
(71) Applicant: GENERAL ELECTRIC COMPANY, Schenectady, NY 12345 (US)
(72) Inventor: Dumoulin, Charles Lucian, Ballston Lake, New York 12019 (US); Darrow, Robert David, Scotia, New York 12302 (US)
(74) Representative: Lupton, Frederick

(57) **Abstract**

RF tracking system employs a RF invasive device (120) coupled to surgical tracking equipment for tracking the invasive device (120). An inductive coupling permits the device to be quickly coupled to, and decoupled from, the equipment. The coupling comprises an inducting coil (260) which transmits a signal from the surgical tracking equipment to a communicating coil (250) in the invasive device (120). The signal received by the communicating coil (250) passes along leads (210) to a tracked coil (200) in a distal end of the invasive device (120). The tracked coil (200) transmits the signal as RF energy which is received by the surgical tracking equipment which superimposes the position of the distal end of the invasive device (120) on an X-ray image (150) and displays it on a monitor (151). A sterile shield (230) is employed as a sterile barrier between the inducting coil (260) and the equipment end of the invasive device (120) to prevent contamination of the invasive device by the inducting coil (260). The cross-section of the invasive device (120) at its equipment end can be made identical to the rest of the invasive device (120) to permit other invasive devices to pass completely over the invasive device (120).

## Description

### BACKGROUND OF THE INVENTION

### 1. Field of the Invention

The present invention relates to medical procedures in which an invasive device is inserted into a living body, and more particularly concerns the tracking of such a device with the use of radiofrequency fields.

### 2. Description of Related Art

X-ray fluoroscopes are used routinely to monitor the placement of invasive devices during diagnostic and therapeutic medical procedures. Conventional X-ray fluoroscopes are designed to minimize X-ray dosage. Nevertheless, some procedures can be very long and the accumulated X-ray dose to the patient can become significant. The long term exposure of the attending medical staff is of even greater concern since they participate in these procedures regularly. Consequently, it is desirable to reduce the X-ray dose during these procedures.

A method to track an end of an invasive device without the use of X-rays has been disclosed previously in European patent application 92307909.9 (RD-20753) hereby incorporated by reference. This application describes a system in which an invasive device incorporating a radiofrequency coil is placed within the body and its position is followed by broadcasting and detecting a radiofrequency (RF) signal.

Typically a connection between an invasive device and the remainder of a tracking system is made by standard physical connections (e.g. BNC or SMD connectors). Invasive devices such as RF catheters and biopsy needles can be constructed with conventional connectors only if such connector is attached to a invasive device exiting the side of the device, since the interior of the device must be available to provide access for guide wires, contrast media and/or any other object which is part of the procedure. A guide wire, on the other hand, is placed within the body prior to the insertion of a catheter. Since the catheter is placed over the exposed end of the guide wire and since it might need to be changed during the procedure, the guide wire must have a connection whose cross-section is smaller than the catheter. One way to avoid this problem is to make the guide wire very long and place the catheter over the guide wire before it is inserted into the patient. Very long guide wires, however, would be cumbersome to use, and changing the catheter with such a system would necessitate the removal of both the guide wire and catheter.

Another important consideration is the maintenance of a sterile instrument and work area during the procedure. In a typical X-ray angiography procedure, the doctor removes the guide wire every few minutes to sterilize it and to remove any thrombus which might have collected. An exceedingly long catheter or guide wire, or one which is difficult to connect and disconnect, would increase the risk of contamination. The sterility of the equipment to which the catheter and guide wire are attached is also important. Any physical contact of the catheter or guide wire with a non-sterile device requires that the device be either re-sterilized or discarded.

### SUMMARY OF THE INVENTION

The present invention provides an electrical connection for connection to surgical tracking equipment comprising:
(a) an invasive device having an equipment end and a distal end, the invasive device having a communicating coil in the equipment end, such that electromagnetic signals may be communicated between the equipment end of the invasive device and the distal end of the invasive device;
(b) a retaining means affixed to said surgical tracking equipment for retaining the equipment end of the invasive device, the retaining means capable of quickly attaching or releasing the invasive device;
(c) an inducting coil connected to said surgical tracking equipment and located adjacent the retaining means, the coil being inductively coupled to the communicating coil in the equipment end of the invasive device; and
(d) a sterile shield situated between the equipment end of the invasive device and the retaining means to prevent contamination of the invasive device.

Invasive devices incorporating RF coils used for tracking the device are inductively coupled to a surgical monitoring or tracking system allowing sterile interfaces between the tracking system equipment and the invasive devices. Connections can therefore be easily broken and reestablished, preserving the sterile nature of each invasive device. Inductive coupling of the device to the surgical tracking system equipment is used so that no direct contact between the device and the tracking system is necessary. The connection comprises an inductive coil connected to the surgical tracking equipment. A retaining means holds an equipment end of the invasive device having a communicating coil which communicates with the inductive coil by electrical induction. The invasive device also has an distal end which is inserted into a patient. Leads running the length of the invasive device conduct a signal to the distal end inside the patient. A disposable sterile shield acts as a sterile barrier between the surgical tracking system equipment and the invasive device to prevent contamination. If necessary, the inductively coupled interface between the invasive device and the equipment is constructed with a small cross-section to permit the invasive device to pass through another invasive. device in the fashion currently employed in diagnostic radiology.

Features of the invention are as follows:
to provide an inductively coupled electrical connection to invasive RF devices; to provide a method of coupling of an invasive device to an external RF system in a manner which maintains a sterile barrier between the device and external system; and to provide a means of rapidly connecting and disconnecting invasive devices to an external system.

### BRIEF DESCRIPTION OF THE DRAWINGS

The features of the invention believed to be novel are set forth with particularity in the appended claims. The invention itself, however, both as to organization and method of operation, together with further objects and advantages thereof, may best be understood by reference to the following description taken in conjunction with the accompanying drawings in which:
Figure 1 is a perspective view of one embodiment of the present invention in use during an invasive surgical procedure.
Figure 2 is a part sectional, part schematic diagram illustrating the inductive coupling between an RF invasive device having an distal end and surgical tracking system equipment for tracking the distal end of the invasive device.
Figure 3 is a perspective view of an invasive device inserted in a subject having a hollow center for receiving other surgical apparatus.

### DETAILED DESCRIPTION OF THE INVENTION

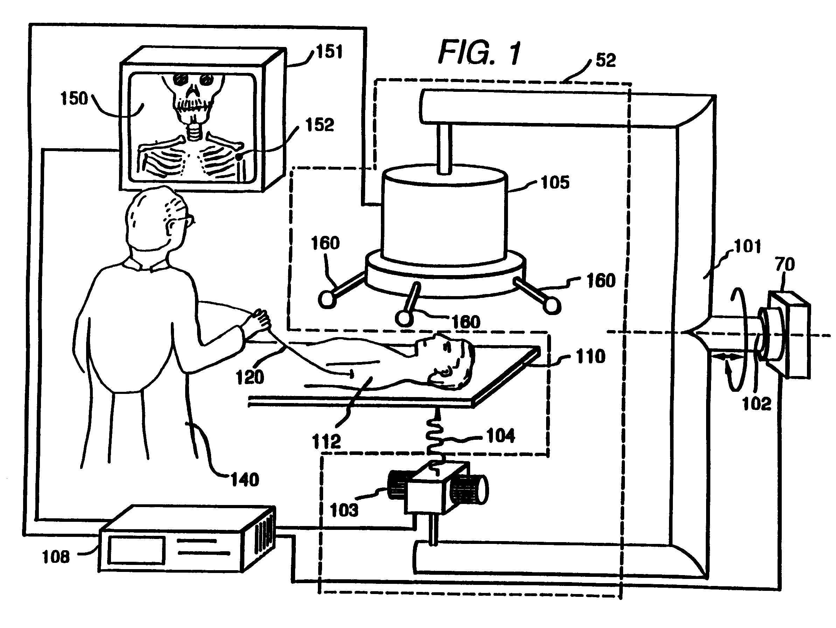

In Figure 1, a distal end of a invasive device 120 is introduced into a living subject 112 and tracked as described in aforementioned European patent application 92307909.9 (RD-20753). A support arm 101, capable of being rotated about at least one axis 102 and translated by a gantry control means 70, is provided in order to hold an X-ray source 103 that emits a substantially collimated beam of X-rays 104 suitable for X-ray imaging and X-ray fluoroscopy. Support arm 101 also holds an X-ray detection means 105 aligned with the propagation direction of X-rays 104 emitted by an X-ray source 103. X-rays 104 penetrate a subject support table 110 and subject 112. An invasive device 120 having an distal end is positioned inside the subject by an operator 140. The location of distal end of invasive device 120 is visible as a superimposed image or symbol 152 on an X-ray image of the subject displayed on a display monitor 151. Display monitor 151 is driven by tracking/display unit 108. In fluoroscopic usage, X-ray images are acquired and displayed several (12 to 60) times a second.

The invasive device 120 shown entering the left arm of the subject incorporates at least one small tracked coil (not shown) which is driven by signals propagated from a device interface assembly 125. Tracking/display unit 108 provides power to the tracked coil to create a dipole electromagnetic field which is detected by RF receive coils 160. The tracked coil located in the distal end of invasive device 120 creates a dipole electromagnetic field. This dipole field induces currents and voltages in an array of receive coils 160 distributed around a region of interest. These voltage signals from receive coils 160 are digitized and sent to tracking computer 108 for analysis. Tracking/display unit 108 utilizes non-linear iterative methods to solve several simultaneous equations describing a dipole field to determine the position and orientation of the tracked coil (and therefore the distal end of invasive device 120) as described in European patent application 92307909.9 (RD-20753). The calculated position of the distal end of invasive device 120 is displayed by superposition of a symbol 152 on an X-ray image appearing on video monitor 151.

Following the preferred procedure, operator 140 initiates acquisition of an X-ray image only when it is deemed necessary, in order to minimize X-ray dose to subject 112 and operator 140. The instantaneous location of the distal end of invasive device 120 is updated several times per second (ideally 12 to 60 times per second to simulate the motion of conventional fluoroscopy systems) and therefore provides an approximation of the fluoroscopic image of the distal end of invasive device 120 that operator 140 would expect to see with a conventional X-ray fluoroscopic system.

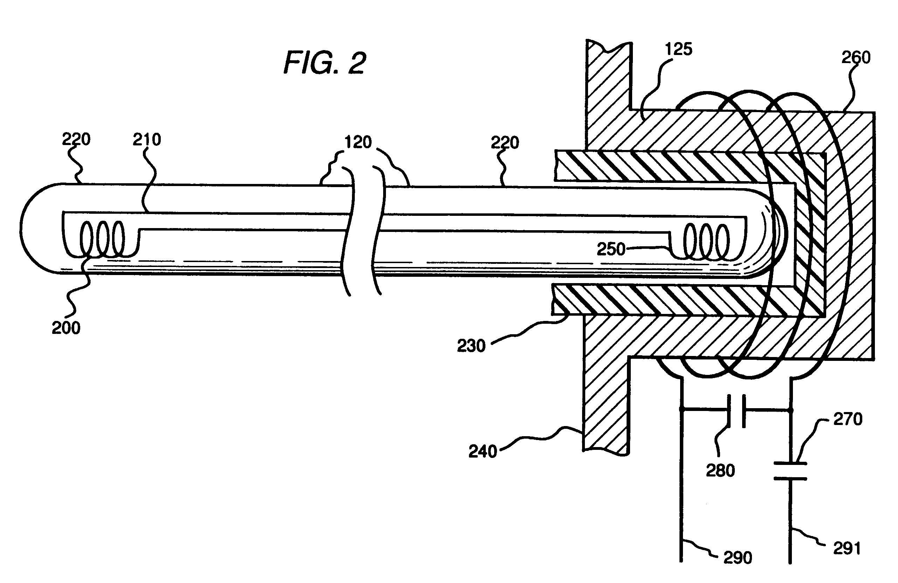

One embodiment of invasive device 120 is shown in greater detail in Fig. 2. The distal end of invasive device is comprised of a tracked coil 200 which is driven by an electrical signal propagated by a pair of conductors 210. This pair of conductors can be arranged either side-by-side, as shown, or coaxially. Tracked coil 200 and conductors 210 are hermetically encased by the exterior surface 220 of the invasive device. The opposite end of the invasive device being the equipment end, is placed in a sterile shield 230 which provides a sterile barrier between the invasive device's exterior surface 220 and exterior surface 240 of interface assembly 125 of the surgical tracking system equipment. A communicating coil 250 within invasive device 120 is inductively coupled to an inductive coil 260 which is tuned to the desired radio frequency using the tuning capacitor 280. Inductive coil 260 is driven by an amplifier (not shown) through conductors 290 and 291. Matching capacitor 270 adjusts the impedance in order to maximize the power transmitted by inductive coil 260. The tracked coil 200 is then tracked as described in European patent application 92307909.9 (RD-20753).

In the embodiment of the invention shown in Fig. 2 the equipment end of invasive device 120 which enters the interface assembly 125 has a diameter which does not exceed that of the rest of the invasive device. This permits larger devices to pass circumferentially over invasive device 120 when invasive device 120 is detached from interface assembly 125. A further aspect of this embodiment is that RF transmit coil 200 and RF communicating coil 250 inside the invasive device 120 are identically constructed and either end of invasive device 120 may be placed in interface assembly 125. An additional aspect is that the axis of communicating coil 250 is coincident with the axis of the equipment end of the invasive device when coupled to interface assembly 125, thereby maximizing the inductive coupling and permitting rotation of invasive device 120 within interface assembly 125. Axes of transmit coil 200 and inductive coil 260 will be co-linear if the distal end of invasive device 120 is inserted in interface assembly 125.

Several means can be used to insure that invasive device 120 fits securely in interface assembly 125. In the embodiment as shown in Fig. 2, friction between the external surface 220 of invasive device 120 and the sterile shield supported by the external surface 240 of interface assembly 125 holds the equipment end of invasive device 120 in place. Other methods of securing invasive device 120 to interface assembly 125 can include means which make use of suction, magnetic attraction or the like.

The use of this invention can apply to guide wires, catheters, endoscopes, laparoscopes, and other surgical instruments, three-dimensional pointers, and therapeutic devices such as those used for RF ablation therapy.

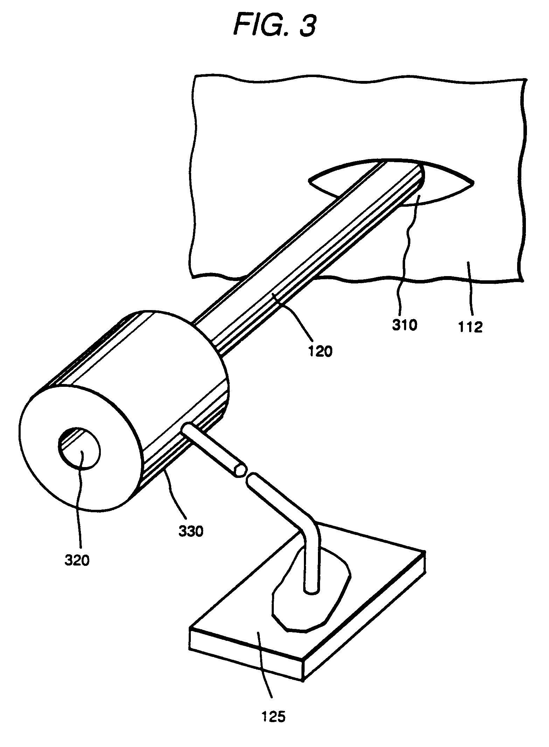

Fig. 3 shows an embodiment of invasive device 120 inserted through incision 310 of subject 112. A center portion 320 of invasive device 120 is free to accept other surgical equipment. Leads 210, 220 travel from interface assembly 125 through the side of invasive device 120 and collar 330. The leads then pass inside incision 310 through invasive device 120.

In the preferred embodiment of the invention, inductive coil 260 transmits radiofrequency energy which is received by communicating coil 250, passes along leads 210 to tracked coil 200 which radiates the energy. However, reciprocity between transmitters and the receivers exists such that tracked coil 200 may receive energy within the subject 112 of Fig. 1 from coils 160, pass the energy via leads 210 to communicating coil 250 that will transmit RF energy and inductive coil 260

It may be advantageous to have multiple coil pairs 200, 250 within invasive device 120 to provide additional information to the tracking system. These multiple coils can be either frequency-multiplexed or time-multiplexed by the tracking system. It may also be advantageous to add additional inductors and capacitors to the coils to match and tune the invasive device to selected frequencies.

While several presently preferred embodiments of the novel inductively coupled RF invasive devices have been described in detail herein, many modifications and variations will now become apparent to those skilled in the art. It is, therefore, to be understood that the appended claims are intended to cover all such modifications and variations.

## Claims

1. An electrical connection for connection to surgical tracking equipment comprising:
a) an invasive device having an equipment end and a distal end, the invasive device having a communicating coil in the equipment end, such that electromagnetic signals may be communicated between the equipment end of the invasive device and the distal end of the invasive device;
b) a retaining means affixed to said surgical tracking equipment for retaining the equipment end of the invasive device, the retaining means capable of quickly attaching or releasing the invasive device;
c) an inducting coil connected to said surgical tracking equipment and located adjacent the retaining means, the coil being inductively coupled to the communicating coil in the equipment end of the invasive device; and
d) a sterile shield situated between the equipment end of the invasive device and the retaining means to prevent contamination of the invasive device.

2. The RF tracking system of claim 1, wherein the invasive device comprises one of the group consisting of a guide wire, a catheter, an endoscope, a laparoscope, a biopsy needle and an RF ablation catheter.

3. The electrical connection of any preceding claim further comprising a tracked coil situated in the distal end of the invasive device and adapted to transmit RF energy into a subject.

4. The electrical connection of any preceding claim wherein the invasive device is constructed with uniform cross-section throughout its length.

5. The electrical connection of any preceding claim wherein the invasive device employs an opening through its length adapted for receiving other invasive devices.

6. The electrical connection of any preceding claim wherein the retaining means retains the equipment end of the invasive device by one of the group consisting of friction, suction and magnetic attraction.

7. The electrical connection of any preceding claim including matching capacitor coupled to said inducting coil to tune the inducting coil to a desired radiofrequency.

8. The electrical connection of any preceding claim wherein the invasive device further comprises a co-axial cable connected between the communicating coil and the tracked coil.

9. The electrical connection of any preceding claim wherein an axis of symmetry of the inducting coil and an axis of symmetry of the communicating coil are substantially co-linear.

10. The electrical connection of any preceding claim wherein an axis of the communicating coil and an axis of symmetry of the equipment end of the invasive device are substantially co-linear.

11. The electrical connection of any preceding claim incorporated in an RF tracking system comprising surgical tracking equipment.
